# FASCICULE DE BREVET EUROPEEN

(11) **EP 2 857 064 B1**
(45) Date de publication et mention de la délivrance du brevet: **14.10.2015**
(21) Numéro de dépôt: 14166139.7
(22) Date de dépôt: 28.04.2014
(51) Int. Cl.: A61N 1/378, H01L 41/113, H02N 2/18

(54) **Capsule intracorporelle autonome à récupération d'énergie par transducteur piézoélectrique**
Autonome intrakorporelle Kapsel mit Energierückgewinnung über einen piezoelektrischen Wandler
Autonomous intracorporeal capsule with energy recovery by piezoelectric transducer

(30) Priorité: 01.10.2013 FR 1359465
(43) Date de publication de la demande: 08.04.2015
(73) Titulaire: Sorin CRM SAS, 92140 Clamart Cedex (FR)
(72) Inventeur: Deterre, Marin, 75013 Paris (FR); Lefeuvre, Elie, 93100 Montreuil (FR)
(74) Mandataire: Dupuis-Latour, Dominique

(56) Documents cités:
- EP-A1- 2 639 845
- WO-A1-2013/077301
- WO-A1-2013/081560
- WO-A1-2013/121759
- US-A- 3 456 134
- US-A1- 2012 286 625

## Description

L'invention concerne, de façon générale, le domaine des "dispositifs médicaux actifs" tels que définis par la directive 93/42/CE du 14 juin 1993 du Conseil des communautés européennes, et notamment les "dispositifs médicaux implantables actifs" tels que définis par la directive du Conseil 90/385/CEE du 20 juin 1990.

Cette définition inclut en particulier les appareils chargés de surveiller l'activité cardiaque et de générer des impulsions de stimulation, de resynchronisation, de défibrillation et/ou de cardioversion en cas de trouble du rythme détecté par l'appareil. Elle inclut aussi les appareils neurologiques, les implants cochléaires, etc., ainsi que les dispositifs de mesure de pH ou encore d'impédance intracorporelle (telle que mesure d'impédance transpulmonaire ou d'impédance intracardiaque).

L'invention concerne plus particulièrement ceux de ces dispositifs qui mettent en oeuvre des capsules autonomes implantées et dépourvues de toute liaison physique à un dispositif principal implanté (tel qu'un boitier de générateur d'impulsions de stimulation).

Ces capsules autonomes sont dénommées pour cette raison "capsules *leadless*", pour les distinguer des électrodes ou des capteurs disposés à l'extrémité distale d'une sonde (*lead*), cette sonde étant parcourue sur toute sa longueur par un ou plusieurs conducteurs reliant par voie galvanique l'électrode ou le capteur à un générateur connecté à l'extrémité opposée, proximale, de la sonde.

De telles capsules *leadless* sont par exemple décrites dans les US 2007/0088397 A1 et WO 2007/047681 A2 (Nanostim, Inc.) ou encore dans le US 2006/0136004 A1 (EBR Systems, Inc.).

Ces capsules *leadless* peuvent être par exemple des capsules épicardiques, fixées à la paroi extérieure du coeur, ou bien des capsules endo-cavitaires, fixées à la paroi intérieure d'une cavité ventriculaire ou auriculaire au moyen d'une vis d'ancrage saillante, prolongeant axialement le corps de la capsule et destinée à pénétrer dans le tissu cardiaque par vissage au site d'implantation. L'invention n'est toutefois pas limitée à un type particulier de capsule, et elle est applicable indifféremment à tout type de capsule *leadless,* quelle que soit sa destination fonctionnelle. Une capsule *leadless* comprend divers circuits électroniques, capteurs, etc. ainsi que des moyens émetteurs/récepteurs de communication sans fil pour l'échange de données à distance.

Dans tous les cas, le traitement des signaux au sein de la capsule et leur transmission à distance nécessite une énergie non négligeable par rapport aux ressources énergétiques que peut stocker cette capsule. Or, compte tenu de son caractère autonome, la capsule ne peut faire appel qu'à ses ressources propres, à savoir un système d'auto-alimentation intégré comprenant un récupérateur d'énergie associé à une petite batterie tampon intégrée.

L'invention concerne plus précisément les capsules dont le récupérateur d'énergie utilise un transducteur mécano-électrique de type piézoélectrique (ci-après "composant piézoélectrique") sollicité de façon cyclique et alternative en flexion de manière à engendrer des charges électriques, charges qui sont ensuite récupérées par le système d'auto-alimentation de la capsule. Cette sollicitation mécanique du composant peut notamment provenir des variations de pression du fluide environnant la capsule (typiquement, le milieu sanguin), qui déforment ou déplacent cycliquement une membrane flexible ou bien une surface mobile liée à un soufflet (organes désignés ci-après "élément d'actionnement"), cette membrane ou cette surface étant reliée au composant piézoélectrique par un organe de couplage approprié tel que tige, biellette, entretoise, etc. (ci-après "élément de liaison").

Le EP 2 639 845 A1 (Sorin CRM) décrit une telle structure de récupérateur d'énergie.

D'autres exemples de récupérateurs d'énergie mettant en oeuvre un composant piézoélectrique sont divulgués par les WO2013/081560 A1, US 3 456 134 A, US2012/286625 A1, WO 2013/121759 A1 ou encore WO 2013/077301 A1.

Deux configurations sont envisageables, selon la manière dont le composant piézoélectrique est monté dans le boitier de la capsule et selon la position du point d'application de la force transmise par l'élément de liaison qui sollicite le composant.

Dans *une première configuration*, non symétrique, le composant piézoélectrique est en forme de lame ou poutre (au sens de la résistance des matériaux) solidarisée au corps de la capsule en une seule de ses extrémités (configuration "encastrée-libre") et qui est sollicitée en flexion par une force appliquée à son extrémité libre, opposée. Cette configuration autorise une déformation maximale de la lame, donc un niveau élevé de charges générées et de ce fait un excellent rendement de conversion mécano-électrique. En revanche, la disposition non symétrique des différents éléments, notamment de l'élément de liaison par rapport au corps, impose un déplacement non symétrique de l'élément d'actionnement et une déformation non homogène de la membrane ou du soufflet par rapport au corps de la capsule, ce qui n'est pas souhaitable pour des raisons de fiabilité mécanique.

Dans *une seconde configuration*, symétrique, le composant piézoélectrique est solidarisé au corps à ses deux extrémités (configuration "encastrée-encastrée") et sollicité en flexion par une force appliquée en son centre. Cette configuration autorise un déplacement de l'élément d'actionnement parallèlement à lui-même et donc une déformation homogène de la membrane ou du soufflet. En revanche, elle ne permet pas une déformation de grande amplitude du composant piézoélectrique, cette seconde configuration étant beaucoup plus rigide que la première : typiquement, pour une même dimension de composant, la raideur d'une configuration encastrée-encastrée est huit fois plus élevée que celle d'une configuration encastrée-libre, conduisant à un déplacement en flexion du composant, et donc à une énergie récupérée, huit fois moindre.

Pour augmenter la flexibilité, il faut soit diminuer l'épaisseur du composant piézoélectrique (mais on atteint rapidement les limites des solutions technologiques), soit augmenter sa longueur tout en gardant la configuration symétrique.

Cette dernière solution est celle proposée par le EP 2 639 845 précité, qui enseigne de structurer le composant avec une forme de spirale ou de serpentin pour en augmenter la longueur effective ainsi que la flexibilité, tout en gardant un couplage centré permettant une déformation symétrique du soufflet ou de la membrane. Cependant, bien que de telles structures soient très flexibles, le rendement de transduction reste relativement faible, en raison de deux phénomènes spécifiques propres :
- les structures courbées ou enroulées sont sujettes à des phénomènes de torsion, qui font qu'une large partie de l'énergie mécanique appliquée au transducteur est stockée sous forme d'énergie élastique de torsion, alors que seule l'énergie de flexion est convertie en électricité ;
- les déformations mécaniques d'une structure allongée et enroulée (et même d'une simple structure rectiligne de type encastrée-encastrée) sont complexes, avec des inflexions de courbure : sous contrainte, le composant présente des zones sous tension alternant avec des zones sous compression, créant des changements de signe du potentiel électrique créés par l'effet piézoélectrique (changements d'autant plus nombreux que la structure est allongée et de forme complexe pour augmenter sa flexibilité). Ce phénomène peut être pris en compte en munissant le composant d'électrodes de récupération des charges qui soient distinctes pour chaque zone respective sollicitée en tension ou en compression, mais cette structuration des électrodes ajoute une complexité supplémentaire de conception et de réalisation du composant, sans pour autant remédier complètement au mauvais rendement de conversion résultant de la multiplication des changements de signe du potentiel électrique au sein du matériau piézoélectrique.

Le but de l'invention est de pallier ces contraintes et limitations, en proposant un nouveau type de récupérateur d'énergie pour capsules *leadless* incorporant un composant piézoélectrique avec un rendement de transduction élevé, permettant de convertir en électricité la plus grande partie possible d'une énergie mécanique d'entrée produite par un élément d'actionnement sollicité cycliquement.

Un autre but de l'invention est de faire en sorte que la sollicitation du composant piézoélectrique, qui résulte typiquement des variations de pression du milieu ambiant telles que les variations de pression du sang au cours de cycles cardiaques successifs, soit associée à une déformation homogène des différents organes de l'élément d'actionnement (soufflet, membrane...), garantissant de ce fait une excellente fiabilité mécanique sur le long terme.

Essentiellement, l'invention propose, dans un tel récupérateur d'énergie, d'utiliser un composant piézoélectrique avec :
- une configuration de type encastré-libre simple, et
- entre l'élément d'actionnement et l'extrémité libre du composant, un couplage mécanique réalisé par un organe disposé sur l'extrémité libre et qui autorise un degré de liberté en pivotement de cette extrémité libre, de manière à permettre la transmission des efforts sans inflexion de courbure du composant, et donc sans effet de réaction qui conduirait à une déformation non homogène du soufflet ou de la membrane de la capsule.

Plus précisément, l'invention propose une capsule intracorporelle autonome comportant, de manière en elle-même connue notamment d'après le EP 2 639 845 A1 précité, un corps et, à l'intérieur de ce corps, des circuits électroniques et des moyens de récupération d'énergie pour l'alimentation électrique de ces circuits électroniques. Les moyens de récupération d'énergie comprennent un élément d'actionnement, un transducteur mécano-électrique comprenant au moins un composant piézoélectrique déformable, un élément de liaison couplant l'élément d'actionnement et le composant piézoélectrique, et des moyens de récupération des charges électriques produites par le composant piézoélectrique. L'élément d'actionnement comprend une surface mobile du corps de la capsule apte à être soumise aux variations cycliques de pression dans le milieu environnant la capsule et à produire une sollicitation mécanique cyclique sous l'effet de ces variations de pression ; l'élément de liaison transmet au composant piézoélectrique, en un point d'application et suivant une direction d'application, cette sollicitation mécanique et produit ainsi cycliquement des déformations de flexion du composant piézoélectrique propres à engendrer des charges électriques, le composant piézoélectrique comportant une lame flexible allongée rectiligne s'étendant suivant une direction principale de lame, perpendiculairement à la direction d'application de la sollicitation mécanique.

De façon caractéristique de l'invention, la lame est configurée en une poutre encastrée-libre apte à être contrainte de manière à fléchir sans inversion de courbure, avec une extrémité encastrée solidaire du corps de la capsule et une extrémité libre, opposée, reliée à l'élément de liaison au point d'application de la sollicitation mécanique. Il est en outre prévu des moyens de couplage de l'extrémité libre de la lame à l'élément de liaison, ces moyens de couplage présentant un degré de liberté en pivotement entre la direction principale de lame et la direction d'application de la sollicitation mécanique.

Selon diverses caractéristiques subsidiaires avantageuses :
- les moyens de couplage comprennent un élément intermédiaire en matériau polymère flexible reliant l'extrémité libre de la lame avec l'élément de liaison ;
- les moyens de couplage comprennent un étrier relié à l'élément de liaison avec deux branches disposées de part et d'autre de l'extrémité libre de la lame et un intercalaire de couplage respectif disposé entre chaque branche et la face en vis-à-vis de la lame. L'intercalaire de couplage peut notamment comprendre un élément en matériau polymère flexible reliant la branche à la face en vis-à-vis de la lame, un pointeau formant point d'appui de la branche contre la face en vis-à-vis de la lame, ou bien un roulement à bille ou à galet formant palier d'appui de la branche contre la face en vis-à-vis de la lame ;
- les moyens de couplage comprennent une articulation avec une rotule solidaire de l'extrémité libre de la lame, coopérant avec un siège formé sur l'élément de liaison, ou *vice versa* ;
- la capsule comprend une structure asymétrique, où l'élément de liaison est relié en un point central de l'élément d'actionnement, ou bien une structure dédoublée symétrique, avec deux composants piézoélectriques et deux éléments de liaison respectifs reliés à l'élément d'actionnement commun en des points diamétralement opposés de cet élément d'actionnement ;
- la lame présente une largeur progressivement décroissante de son extrémité encastrée vers son extrémité libre.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 illustre de façon schématique un ensemble de dispositifs médicaux comprenant notamment des capsules *leadless,* implantées au sein du corps d'un patient.
La Figure 2 est un schéma par blocs fonctionnels montrant les différents étages constitutifs d'une capsule *leadless.*
Les Figures 3 et 4 illustrent deux formes de réalisation possibles du corps d'une capsule *leadless* à récupération des variations de pression du fluide environnant.
Les Figures 5a, 5b et 5c sont des vues, respectivement en coupe au repos, en coupe en fonctionnement, et en perspective schématique, d'un récupérateur d'énergie à transducteur piézoélectrique selon l'état de la technique.
Les Figures 6 et 7 sont des vues de dessus de composants piézoélectriques formés de bandes enroulées en serpentin ou en spirale, selon l'état de la technique.
La Figure 8 est une vue schématique en perspective illustrant les déformations subies par un composant piézoélectrique de type encastré-encastré.
Les Figures 9a, 9b et 9c sont des vues en coupe, respectivement au repos, en fonctionnement avec une sollicitation dans un sens et en fonctionnement avec une sollicitation dans le sens opposé, d'un système de récupération d'énergie selon un premier mode de réalisation de l'invention.
La Figure 10 est homologue de la Figure 9b, pour une variante du premier mode de réalisation avec dédoublement du transducteur piézoélectrique.
Les Figures 11a, 11b et 11c sont des vues de dessus de différentes formes possibles de composant piézoélectrique dédoublé, utilisable notamment avec le mode de réalisation de la Figure 10.
Les Figures 12a, 12b et 12c sont homologues des Figures 9a, 9b et 9c, pour un deuxième mode de réalisation de l'invention.
Les Figures 13a, 13b et 13c sont homologues des Figures 9a, 9b et 9c, pour un troisième mode de réalisation de l'invention.
Les Figures 14a, 14b et 14c sont homologues des Figures 9a, 9b et 9c, pour un quatrième mode de réalisation de l'invention.
Les Figures 15a, 15b et 15c sont homologues des Figures 9a, 9b et 9c, pour un cinquième mode de réalisation de l'invention.

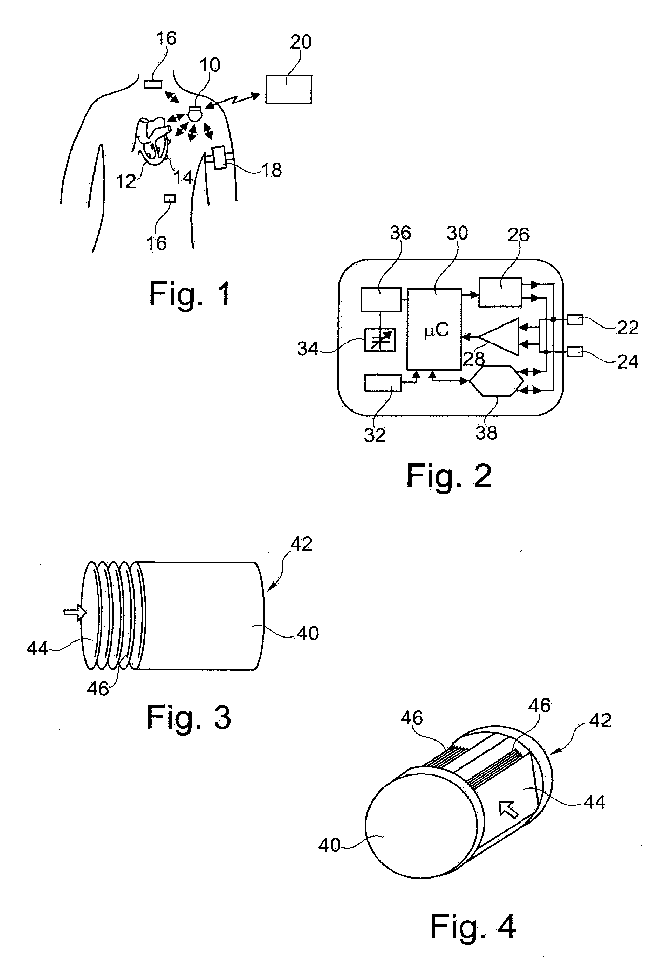

On va tout d'abord décrire, en référence aux Figures 1 à 8, une structure connue de capsule *leadless,* avec un exemple de réalisation d'une telle capsule *leadless* à récupérateur d'énergie à composant piézoélectrique selon l'état de la technique.

Sur la Figure 1 on a illustré un ensemble de dispositifs médicaux implantés au sein du corps d'un patient.

Celui-ci est équipé par exemple d'un implant 10 tel qu'un défibrillateur/ stimulateur/resynchroniseur implanté, un défibrillateur sous-cutané ou encore un enregistreur longue durée. Ce dispositif 10 est le dispositif maitre d'un réseau comportant une pluralité de dispositifs esclaves 12 à 18, qui peuvent notamment inclure des capsules intracardiaques 12 ou épicardiques 14 implantées directement sur le coeur du patient, d'autres dispositifs 16 tels que capteurs de myopotentiels ou dispositifs de stimulation neurologique, et éventuellement un dispositif externe 18 disposé sur un brassard et pourvu d'électrodes en contact avec la peau. Le dispositif 10 peut également être utilisé en tant que passerelle avec le monde extérieur pour communiquer avec un périphérique externe 20 de type programmateur ou dispositif de télétransmission de données avec lequel il pourra communiquer par télémétrie.

La Figure 2 illustre de façon schématique les différents circuits internes des capsules autonomes implantées 12 à 16.

La capsule comporte par exemple un couple d'électrodes 22, 24 reliées à un circuit 26 générateur d'impulsions de stimulation (pour une capsule active incorporant cette fonction) et/ou à un circuit de détection 28 servant au recueil des potentiels de dépolarisation recueillis entre les électrodes 22 et 24. Un circuit central 30 inclut l'ensemble de l'électronique permettant de piloter les diverses fonctions de la capsule, la mémorisation des signaux recueillis, etc. Il comprend un microcontrôleur et un oscillateur générant les signaux d'horloge nécessaires au fonctionnement du microcontrôleur et à la communication. Il peut également contenir un convertisseur analogique/numérique et une mémoire de stockage numérique. La capsule peut également être pourvue d'un capteur 32 tel qu'un capteur d'accélération, de pression, un capteur hémodynamique, de température, de saturation en oxygène, etc. La capsule comprend un module de récupération d'énergie 34 alimentant l'ensemble des circuits via un étage de gestion d'énergie 36. Les électrodes 22 et 24 sont également reliées à un circuit d'émission/réception d'impulsions 38 servant à la communication sans fil avec le dispositif maitre ou les autres capsules.

L'invention concerne plus particulièrement le module de récupération d'énergie 34.

Il s'agit de récupérer l'énergie contenue dans des efforts mécaniques auxquels est soumise la capsule, plus précisément de récupérer l'énergie des variations de pression du fluide dans lequel baigne la capsule, typiquement les variations de pression sanguine.

Pour prendre en compte ces variations, la capsule est réalisée, comme illustré Figures 3 et 4, avec un corps 40 pourvu d'un ou plusieurs éléments déformables sollicités au rythme de ces variations de pression, avec une surface rigide 44, sur laquelle s'exerce la pression, qui est reliée au reste du corps 40 par un soufflet déformable 46. Dans l'exemple de la Figure 3, l'ensemble surface/soufflet 44/46 est disposé sur une face d'extrémité axiale de la capsule 40, tandis que dans l'exemple de la Figure 4 il est prévu deux ensembles surface/soufflet 44/46 disposés sur des faces latérales du corps 40 de la capsule, les surfaces rigides 44 étant parallèles entre elles et à l'axe principal de la capsule.

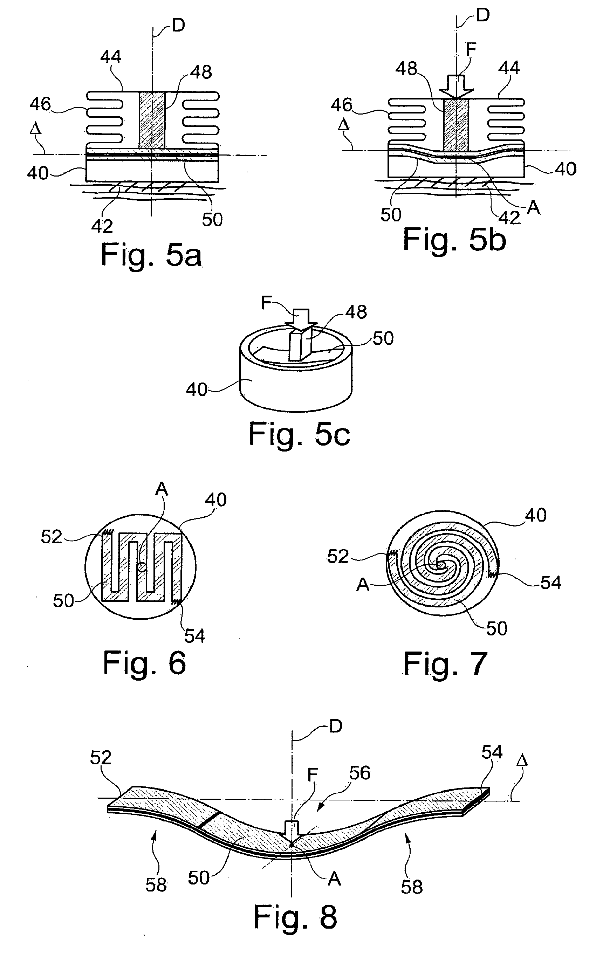

La capsule porte, sur sa face destinée à venir en contact avec la paroi corporelle, des moyens d'accrochage 42 (schématisés notamment Figures 5a et 5b) tels que vis ou barbes permettant d'ancrer la capsule à l'endroit du site d'implantation choisi, par exemple sur une paroi intérieure d'une cavité du myocarde.

Les Figures 5a à 5c illustrent de façon schématique une configuration de transducteur piézoélectrique selon l'état de la technique, pour un récupérateur à soufflet tel que celui illustré Figures 3 et 4 et divulgué par exemple dans le EP 2 520 333 A1 (Sorin CRM).

Sur cet exemple de réalisation connu, la sollicitation physique extérieure F, qui résulte des variations de la pression sanguine subie par la surface rigide 44, est transmise via une tige 48 ou élément de liaison analogue à un composant 50 formant transducteur de type piézoélectrique de récupération d'énergie. Ce composant 50 permet de convertir la sollicitation mécanique F en charges électriques grâce à l'effet piézoélectrique direct, selon lequel la sollicitation mécanique F transmise par l'élément de liaison 48 engendre des charges électriques récupérées par des électrodes formées à la surface du composant piézoélectrique 50. L'énergie électrique ainsi récupérée est ensuite traitée par le module 36 de stockage et de gestion d'énergie.

Du point de vue dimensionnel, les structures de composant piézoélectrique utilisées font quelques millimètres de longueur, quelques centaines de micromètres à quelques millimètres de largeur, et quelques dizaines à quelques centaines de micromètres d'épaisseur. En ce qui concerne le matériau, les couches piézoélectriques du composant 50 peuvent être réalisées en un matériau tel qu'une céramique PZT ou des monocristaux de type PMN-PT, titanate de baryum ou niobate de lithium présentant un fort couplage électromécanique.

De façon générale, l'énergie mécanique en entrée due à la force de pression sanguine est de faible intensité, de quelques dizaines à quelques centaines de mN pour un déplacement de l'ordre de quelques centaines de µm. Ceci implique que la raideur du système soit faible, typiquement quelques centaines à quelques milliers de mN/m.

Pour répondre à ce critère de flexibilité (faible raideur) tout en restant compatible avec l'exigence de miniaturisation, il a été proposé, notamment par le EP 2 639 845 précité, de conformer le composant 50 avec une structure en bande repliée en zigzag ou en spirale.

Cette configuration est illustrée Figures 6 et 7 : le composant 50 en forme de bande est solidarisé au corps 40 de la capsule à ses deux extrémités 52, 54, de manière à obtenir une structure de type "poutre encastrée-encastrée" fine et longue, la sollicitation étant appliquée au point central A. Cet agencement permet d'allonger la longueur de la structure piézoélectrique sans perdre en compacité. Une telle structure présente cependant l'inconvénient de voir apparaitre des phénomènes de torsion, inhérents aux configurations enroulées ou repliées. De ce fait, une part importante de l'énergie mécanique appliquée en entrée est stockée sous forme d'énergie élastique de torsion qui, à la différence de l'énergie de flexion, n'est pas convertible en électricité, dégradant ainsi le rendement de transduction d'énergie mécanique en énergie électrique par rapport à un composant de type poutre rectiligne, qui n'est soumis qu'à des efforts de flexion.

Un autre inconvénient de cette configuration connue tient au fait que, comme illustré Figure 8, dans une structure de poutre "encastrée-encastrée" où le composant piézoélectrique 50 est rigidement fixé à ses deux extrémités 52, 54 au corps de la capsule, la déformation de la poutre va nécessairement provoquer la formation d'une région centrale concave 56 avec de part et d'autre deux régions convexes 58, ou *vice versa* pour une sollicitation dans l'autre sens. Cette inflexion de courbure se traduira électriquement par une inversion de polarité des charges générées le long de la poutre, imposant l'isolement électrique des électrodes en fonction de leur concavité : en effet, pour éviter la recombinaison des charges, les électrodes doivent être séparées sur les différentes régions subissant des courbures différentes. Il est donc nécessaire de structurer les électrodes en jeux distincts, générant chacun une polarité différente. Dans le cas de structures repliées ou enroulées telles que celles des Figures 6 et 7, ces changements de signe sont multipliés, conduisant à des nombreux changements de signes des charges générées qui conduisent à une dégradation du rendement de transduction.

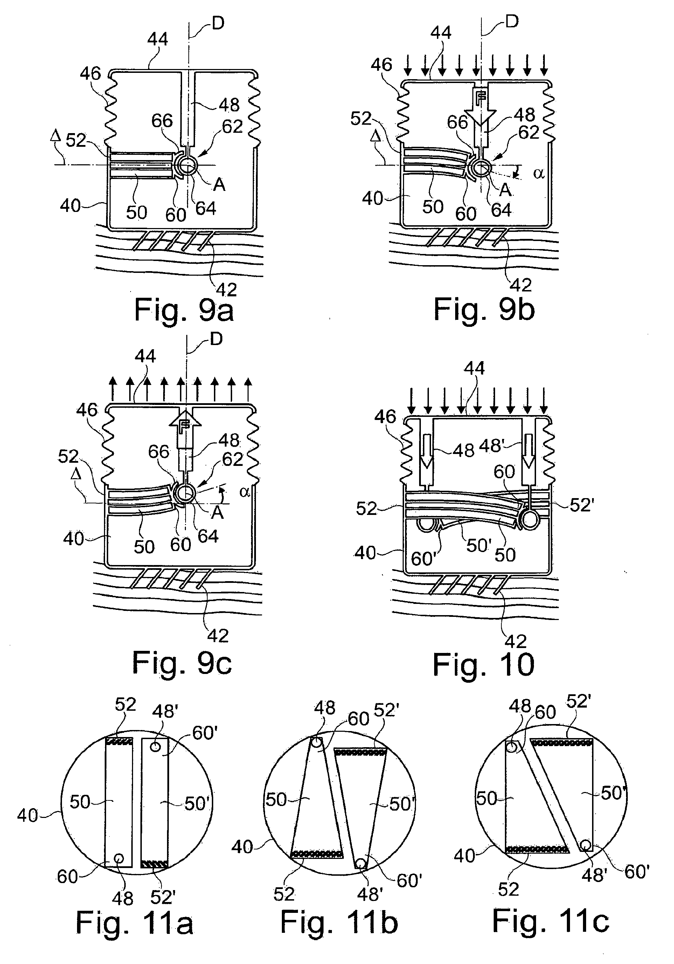

On va maintenant décrire la proposition de l'invention pour remédier à ces divers inconvénients, en référence aux Figures 9 à 15.

Sur ces figures, on a présenté divers modes de réalisation de l'invention permettant d'augmenter très sensiblement le rendement de conversion des dispositifs connus tels que ceux que l'on vient de décrire.

L'idée de base de l'invention consiste, à la différence des configurations "encastrée-encastrée" proposées jusqu'à présent, d'utiliser une simple configuration "encastrée-libre" avec un composant piézoélectrique en forme de lame ou poutre rectiligne.

On a en effet exposé plus haut les avantages procurés en termes de rendement de conversion par une telle structure simplifiée : pas ou peu de contraintes de torsion, pas d'inflexion de courbure susceptible d'inverser la polarité des charges le long d'une même face du composant, faible raideur permettant de maximiser le déplacement lors de la flexion et donc la quantité de charges générées pour un même déplacement de l'élément d'actionnement.

Utilisée telle quelle, ce type de structure "encastré-libre" présenterait l'inconvénient mécanique, du fait des effets de réaction entre le composant piézoélectrique et l'élément d'actionnement, de conduire à un déplacement de l'élément d'actionnement non parallèlement à lui-même, et donc à une déformation non homogène du soufflet (qui s'écraserait plus du côté de l'extrémité libre que de l'extrémité encastrée du composant), entrainant des problèmes majeurs de fiabilité mécanique à long terme de la structure.

Pour éviter cet inconvénient, et permettre une déformation homogène et symétrique du soufflet, l'invention propose, comme on peut le voir notamment sur la Figure 9a, de réaliser une liaison mécanique de type pivot 62 entre, d'une part, l'élément de liaison 48 lié à l'élément d'actionnement 44 recevant les efforts et transmettant ceux-ci suivant la direction D formant axe de symétrie du soufflet 46 et, d'autre part, l'extrémité libre 60 du composant piézoélectrique déformable 50. Ainsi, un tel système n'affecte ni la déformation du soufflet 46 ni le déplacement de l'élément d'actionnement 44, qui pourra se déplacer parallèlement à lui-même, comme illustré Figures 9b et 9c, respectivement dans un sens ou dans l'autre en fonction des variations, positives ou négatives, de la pression environnante. Le composant piézoélectrique 50 pourra fléchir dans un sens ou dans l'autre (Figures 9b et 9c) de part et d'autre de sa position d'équilibre (Figure 9a) correspondant à la direction principale Δ le long de laquelle le composant s'étend. Ces mouvements se feront sans transmettre d'efforts de torsion à l'élément de liaison 48, efforts qui pourraient influer, par réaction, sur le déplacement de l'élément d'actionnement 44 et donc sur la déformation du soufflet 46.

Ceci est obtenu grâce au degré de liberté en pivotement (angle α sur les Figures 9b et 9c), permis par la liaison mécanique de type pivot 62, entre l'extrémité 60 libre du composant 50 et l'élément de liaison 48.

Sur les Figures 9a à 9c, cette liaison a été illustrée sous forme d'une rotule 64 solidaire de l'élément de liaison 48 et coopérant avec un siège 66 solidaire de l'extrémité libre 60 du composant piézoélectrique 50.

Cette configuration présente trois avantages majeurs :
- par rapport à une configuration de type "encastrée-encastrée" (comme sur la Figure 8), la configuration "encastrée-libre" présente une raideur huit fois plus faible ce qui permet, pour une même force F appliquée en entrée (dont la valeur est imposée par l'importance des variations de la pression sanguine et par l'aire de la surface mobile 44), d'augmenter d'un facteur huit l'amplitude du déplacement et donc l'énergie récupérable.
- en second lieu, la structure "encastrée-libre" proposée ne présente qu'un seul type de contrainte mécanique par couche piézoélectrique, du fait de l'absence d'inversion de courbure : uniquement de la tension sur une face, et uniquement de la compression sur la face opposée. Ainsi, une simple électrode non structurée suffira pour récupérer l'énergie, ce qui affranchit la fabrication d'une étape de structuration des électrodes et, surtout, permet de générer une quantité de charges et donc une énergie bien plus grande. Le rendement du transducteur piézoélectrique est par conséquent beaucoup plus élevé qu'avec les structures conventionnelles de l'état de la technique ;
- enfin, une configuration "encastrée-encastrée", en plus d'avoir une raideur huit fois plus élevée qu'une configuration "encastrée-libre", a un comportement très rapidement non-linéaire (dès lors que le déplacement est d'environ la moitié de l'épaisseur de la poutre), ce qui rend cette configuration de fait encore plus rigide.

La Figure 10 illustre une configuration où le composant piézoélectrique 50 occupe le maximum de la longueur permise diamétralement dans le corps 40. Il est en effet intéressant de maximiser la longueur de lame du composant 50 afin de diminuer sa rigidité.

Dans ce cas, pour assurer une déformation symétrique du soufflet 46, il est prévu deux transducteurs identiques 50, 50' configurés tête-bêche, symétriquement par rapport au centre de la capsule. Chaque transducteur 50, 50' possède une extrémité fixe encastrée 52, 52' et une extrémité libre 60, 60' à laquelle est relié un élément de liaison respectif 48, 48' relié à l'élément d'actionnement commun 44 en deux points diamétralement opposés de ce dernier. Les extrémités encastrées 52, 52' sont également situées en deux points diamétralement opposés du corps 40 de la capsule, de sorte que les forces appliquées sur les extrémités libres respectives 60, 60' sont symétriques par rapport à l'axe central de la capsule. La somme des deux forces est par conséquent centrée, ce qui conduit à une déformation symétrique du soufflet 46.

En passant de la configuration de la Figure 9b à celle de la Figure 10 avec des composants piézoélectriques 50, 50' de section identique, le fait i) de pratiquement doubler la longueur de chaque composant 50, 50' et ii) de dédoubler le nombre de composants, permet d'augmenter la flexibilité d'un facteur quatre. De la sorte, le déplacement sera quatre fois plus important pour une force (et donc une variation de pression) donnée, ce qui donnera une énergie elle aussi quatre fois plus importante.

Au final, entre la structure de l'état de la technique telle que celle illustrée Figure 8 et la structure selon l'invention à double composant telle que celle illustrée Figure 10, l'énergie délivrée est augmentée d'un facteur trente-deux.

Les vues en plan des Figures 11a, 11 b et 11 c représentent trois variantes respectives possibles d'optimisation de la forme des poutres de la configuration de la Figure 10, avec des composants 50, 50' de largeur constante (Figure 11a) ou bien de largeur variable, décroissante de l'extrémité fixe encastrée 52, 52' jusqu'à l'extrémité libre 60, 60' (Figures 11b et 11c).

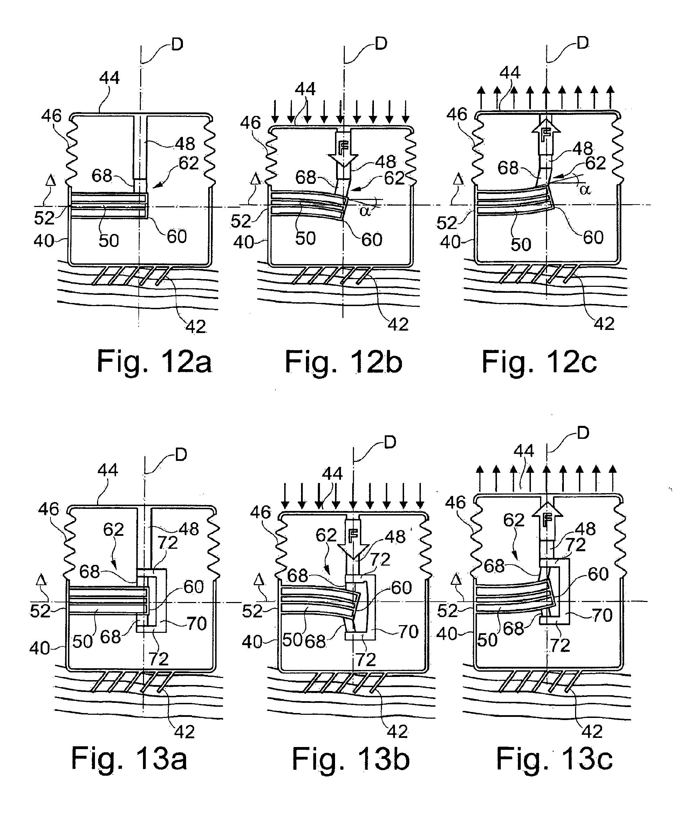

Les Figures 12a à 12c sont homologues des Figures 9a à 9c, pour un mode de réalisation particulièrement avantageux de la liaison à pivot 62.

Dans cette réalisation, la liaison est réalisée à partir d'un simple élément déformable, tel qu'une partie en polymère souple 68 reliant entre elles d'une part l'extrémité libre 60 du composant 50 et d'autre part l'extrémité en vis-à-vis de l'élément de liaison 48. Le polymère 68 peut être un silicone, notamment à base de PDMS (polydiméthylsiloxane), du PEEK (polyétheréthercétone), ou du parylène (poly(p-xylylène)). Lorsque l'élément d'actionnement 44 s'abaisse (Figure 12b) le polymère 68 se compresse et fléchit ; inversement, pour un mouvement dans le sens opposé (Figure 12c) le polymère s'allonge et absorbe la déformation angulaire de l'extrémité libre 60 du composant piézoélectrique 50. Pendant ces mouvements, la surface de contact entre le polymère 68 et l'extrémité libre 60 du composant piézoélectrique 50 ne sera pas horizontale et formera un angle α non nul par rapport à la direction principale Δ du composant 50. Cet angle autorise la déformation du composant 50 de manière arquée, comme dans le cas décrit précédemment aux Figures 9a à 9c, avec l'avantage que cet élément flexible 68 permet de s'affranchir de tout frottement mécanique.

Il est possible suivant le même principe, comme illustré Figures 13a à 13c, de disposer deux de ces éléments flexibles polymère 68 de chaque côté de l'extrémité libre 60 du composant 50, l'un des éléments étant situé sur la surface supérieure du composant et l'autre dans la continuité du premier mais sur la surface inférieure. Ces deux éléments polymère 68 sont reliés aux deux branches 72 d'un étrier 70 lui-même solidaire de l'élément de liaison 48. Lorsque l'élément d'actionnement 44 s'abaisse (Figure 13b), les deux éléments polymère 68 vont se déformer : celui de la surface supérieure va se comporter comme dans le cas de la Figure 12b en mode compression, tandis que celui du bas va se comporter en mode élongation, de façon symétrique pour permettre le pivotement de l'extrémité libre 60 par rapport à la direction D d'application de l'effort F. Dans le déplacement inverse (Figure 13c), les rôles des deux éléments polymère 68 sont inversés, ce qui permet d'avoir globalement un comportement homogène du matériau de ces éléments pour les deux sens de déplacement.

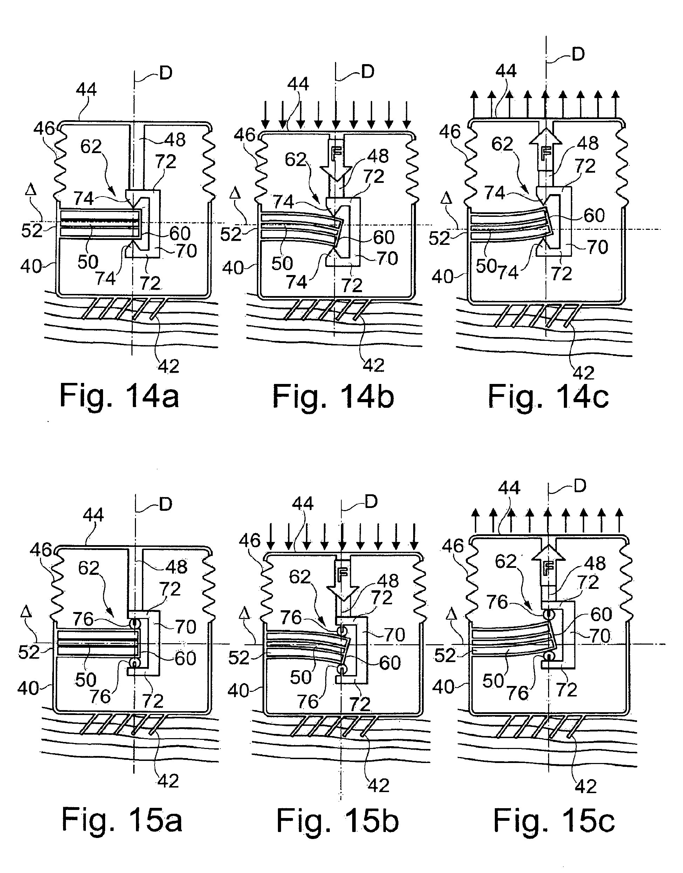

Les Figures 14a à 14c sont homologues des Figures 13a à 13c pour une autre réalisation de la liaison de type pivot 62.

Dans ce cas, le couplage consiste en un simple contact mécanique ponctuel par un pointeau 74 ou élément analogue prévu sur chacune des branches 72 de l'étrier 70 et venant en appui sur le composant 50 de part et d'autre de l'extrémité libre 60. Cette extrémité peut donc être, selon le sens du déplacement de l'élément de liaison 48, poussée (Figure 14b) ou tirée (Figure 14c).

Les Figures 15a à 15c sont homologues des Figures 13a à 13c pour encore une autre réalisation de la liaison de type pivot 62.

Cette réalisation consiste à mettre en place un roulement à bille ou à rouleau 76 entre chaque face du composant et la branche 72 en regard de l'étrier 70. Ainsi, lors des déplacements de l'élément d'actionnement dans un sens ou dans l'autre (Figures 15b et 15c), le mécanisme comportant ces roulements s'abaissera ou se relèvera et se déplacera sur la surface du composant 50 le long de sa direction principale Δ, permettant ainsi à l'extrémité libre 60 de s'incliner et de former un angle sans inflexion de courbure et donc avec une polarité des charges homogène sur une même surface du composant piézoélectrique 50.

## Revendications

1. Une capsule intracorporelle autonome comportant un corps (40) et, à l'intérieur de ce corps, des circuits électroniques (26-38) et des moyens de récupération d'énergie (34) pour l'alimentation électrique de ces circuits électroniques,
les moyens de récupération d'énergie (34) comprenant :
- un élément d'actionnement (44), comprenant une surface mobile du corps de la capsule apte à être soumise aux variations cycliques de pression dans le milieu environnant la capsule et à produire une sollicitation mécanique (F) cyclique sous l'effet de ces variations de pression ;
- un transducteur mécano-électrique comprenant au moins un composant piézoélectrique déformable (50) ;
- un élément de liaison (48), couplant l'élément d'actionnement et le composant piézoélectrique de manière à transmettre au composant piézoélectrique, en un point d'application (A) et suivant une direction d'application (D), la sollicitation mécanique et produire ainsi cycliquement des déformations de flexion du composant piézoélectrique propres à engendrer des charges électriques, le composant piézoélectrique comportant une lame flexible allongée rectiligne (50) s'étendant suivant une direction principale de lame (Δ), perpendiculairement à la direction d'application (D) de la sollicitation mécanique ; et
- des moyens de récupération des charges électriques ainsi produites,
**caractérisé en ce que** :
- la lame est configurée en une poutre encastrée-libre apte à être contrainte de manière à fléchir sans inversion de courbure, avec une extrémité encastrée (52) solidaire du corps (40) de la capsule et une extrémité libre (60), opposée, reliée à l'élément de liaison (48) au point d'application (A) de la sollicitation mécanique ; et
- il est en outre prévu des moyens (62) de couplage de l'extrémité libre de la lame à l'élément de liaison, ces moyens de couplage présentant un degré de liberté en pivotement (α) entre la direction principale de lame (Δ) et la direction d'application (D) de la sollicitation mécanique.

2. La capsule de la revendication 1, dans laquelle les moyens de couplage (62) comprennent un élément intermédiaire en matériau polymère flexible (68) reliant l'extrémité libre de la lame avec l'élément de liaison.

3. La capsule de la revendication 1, dans laquelle les moyens de couplage (62) comprennent un étrier (70) relié à l'élément de liaison avec deux branches (72) disposées de part et d'autre de l'extrémité libre (60) de la lame et un intercalaire de couplage respectif disposé entre chaque branche et la face en vis-à-vis de la lame.

4. La capsule de la revendication 3, dans laquelle l'intercalaire de couplage comprend un élément en matériau polymère flexible (68) reliant la branche à la face en vis-à-vis de la lame.

5. La capsule de la revendication 3, dans laquelle l'intercalaire de couplage comprend un pointeau (74) formant point d'appui de la branche contre la face en vis-à-vis de la lame.

6. La capsule de la revendication 3, dans laquelle l'intercalaire de couplage comprend un roulement (76) à bille ou à galet formant palier d'appui de la branche contre la face en vis-à-vis de la lame.

7. La capsule de la revendication 1, dans laquelle les moyens de couplage (62) comprennent une articulation avec une rotule (64) solidaire de l'extrémité libre de la lame, coopérant avec un siège (66) formé sur l'élément de liaison, ou *vice versa*.

8. La capsule de la revendication 1, comprenant une structure asymétrique, où l'élément de liaison (48) est relié en un point central de l'élément d'actionnement (44).

9. La capsule de la revendication 1, comprenant une structure dédoublée symétrique, avec deux composants piézoélectriques (50, 50') et deux éléments de liaison respectifs (48, 48') reliés à l'élément d'actionnement commun (44) en des points diamétralement opposés de cet élément d'actionnement.

10. La capsule de la revendication 1, dans laquelle la lame (50) présente une largeur progressivement décroissante de son extrémité encastrée (52) vers son extrémité libre (60).

## Patentansprüche

1. Autonome intrakorporelle Kapsel, umfassend einen Körper (40) und im Inneren dieses Körpers elektronische Schaltungen (26-38) und Mittel zur Energierückgewinnung (34) für die elektrische Versorgung dieser elektronischen Schaltungen,
wobei die Mittel zur Energierückgewinnung (34) umfassen:
- ein Betätigungselement (44), umfassend eine mobile Fläche des Körpers der Kapsel, die geeignet ist, den zyklischen Druckschwankungen in dem die Kapsel umgebenden Bereich ausgesetzt zu werden und eine zyklische mechanische Belastung (F) unter der Wirkung dieser Druckschwankungen zu erzeugen;
- einen mechanisch-elektrischen Wandler, mindestens umfassend eine verformbare piezoelektrische Komponente (50);
- ein Verbindungselement (48), das das Betätigungselement und die piezoelektrische Komponente derart koppelt, dass auf die piezoelektrische Komponente an einem Anwendungspunkt (A) und entlang einer Anwendungsrichtung (D) die mechanische Belastung übertragen wird und auf diese Weise zyklisch Biegeverformungen der piezoelektrischen Komponente erzeugt werden, die geeignet sind, elektrische Ladungen zu erzeugen, wobei die piezoelektrische Komponente eine gerades längliches biegsames Blättchen (50) umfasst, das sich in eine Hauptrichtung des Blättchens (Δ) senkrecht auf die Anwendungsrichtung (D) der mechanischen Belastung erstreckt; und
- Mittel zur Rückgewinnung der auf diese Weise erzeugten elektrischen Ladungen,
**dadurch gekennzeichnet, dass**:
- das Blättchen ferner als ein eingesetzterfreier Träger ausgeführt ist, der geeignet ist, derart beansprucht zu werden, dass er sich ohne Krümmungsumkehr biegt, mit einem mit dem Körper (40) der Kapsel verbundenen eingesetzten Ende (52) und einem gegenüberliegenden freien Ende (60), das mit dem Verbindungselement (48) am Anwendungspunkt (A) der mechanischen Belastung verbunden ist; und
- ferner Mittel (62) zur Kopplung des freien Endes des Blättchens mit dem Verbindungselement vorgesehen sind, wobei diese Kopplungsmittel einen Schwenkfreiheitsgrad (α) zwischen der Hauptrichtung des Blättchens (Δ) und der Anwendungsrichtung (D) der mechanischen Belastung aufweisen.

2. Kapsel nach Anspruch 1, bei der die Kopplungsmittel (62) ein Zwischenelement aus einem biegsamen Polymermaterial (68) umfassen, das das freie Ende des Blättchens mit dem Verbindungselement verbindet.

3. Kapsel nach Anspruch 1, bei der die Kopplungsmittel (62) einen Bügel (70) umfassen, der mit dem Verbindungselement verbunden ist, mit zwei Schenkeln (72), die beiderseits des freien Endes (60) des Blättchens angeordnet sind, und einem Zwischenstück zur jeweiligen Kopplung, das zwischen jedem Schenkel und der Fläche gegenüber dem Blättchen angeordnet ist.

4. Kapsel nach Anspruch 3, bei der das Kopplungszwischenstück ein Element aus biegsamem Polymermaterial (68) umfasst, das den Schenkel mit der Fläche gegenüber dem Blättchen verbindet.

5. Kapsel nach Anspruch 3, bei der das Kopplungszwischenstück einen Stift (74) umfasst, der einen Stützpunkt des Schenkels an der Fläche gegenüber dem Blättchen bildet.

6. Kapsel nach Anspruch 3, bei der das Kopplungszwischenstück ein Kugel- oder Rollenlager (76) umfasst, das ein Stützlager des Schenkels an der Fläche gegenüber dem Blättchen bildet.

7. Kapsel nach Anspruch 1, bei der die Kopplungsmittel (62) ein Gelenk mit einer Kugel (64) umfassen, das mit dem freien Ende des Blättchens verbunden ist und mit einem auf dem Verbindungselement ausgebildeten Sitz (66) zusammenwirkt oder umgekehrt.

8. Kapsel nach Anspruch 1, umfassend eine asymmetrische Struktur, bei der das Verbindungselement (48) an einem zentralen Punkt des Betätigungselements (44) verbunden ist.

9. Kapsel nach Anspruch 1, umfassend eine symmetrische Doppelstruktur mit zwei piezoelektrischen Komponenten (50, 50') und zwei jeweiligen Verbindungselementen (48, 48'), die mit dem gemeinsamen Betätigungselement (44) an diametral gegenüberliegenden Punkten dieses Betätigungselements verbunden sind.

10. Kapsel nach Anspruch 1, bei der das Blättchen (50) eine progressiv von seinem eingesetzten Ende (52) zu seinem freien Ende (60) abnehmende Breite aufweist.

## Claims

1. An autonomous intracorporeal capsule, including a body (40) and, inside this body, electronic circuits (26-38) and energy harvesting means (34) for the power supply of these electronic circuits,
the energy harvesting means (34) comprising:
- an actuation element (44), comprising a mobile surface of the body of the capsule adapted to subjected to the cyclic variations of pressure in the environment surrounding the capsule and to produce a cyclic mechanical stress (F) under the effect of these variations of pressure;
- a mechano-electric transducer comprising at least one deformable piezoelectric component (50);
- a link element (48), coupling the actuation element and the piezoelectric component so as to transmit the mechanical stress to the piezoelectric component, at one point of application (A) and following a direction of application (D), and to hence produce cyclically bending deformations of the piezoelectric component liable to generate electric charges, the piezoelectric component including a rectilinear, elongated flexible plate (50) extending in a main plate direction (Δ), perpendicular to the direction of application (D) of the mechanical stress; and
- means for harvesting the so-produced electric charges,
**characterized in that**:
- the plate is configured as a free-embedded beam adapted to be stressed so as to bent with no inversion of curvature, with an embedded end (52) integral with the capsule body (40) and an opposite, free end (60), connected to the link element (48) at the point of application (A) of the mechanical stress; and
- means (62) for coupling the free end of the plate to the link element are further provided, these coupling means having a pivoting degree of freedom (α) between the main plate direction (Δ) and the direction of application (D) of the mechanical stress.

2. The capsule of claim 1, wherein the coupling means (62) comprise a flexible polymeric-material intermediate element (68) connecting the free end of the plate to the link element.

3. The capsule of claim 1, wherein the coupling means (62) comprise a stirrup (70) connected to the link element with two arms (72) arranged on either side of the free end (60) of the plate and a respective coupling insert arranged between each arm and the opposite face of the plate.

4. The capsule of claim 3, wherein the coupling insert comprises a flexible polymeric-material element (68) connecting the arm to the opposite face of the plate.

5. The capsule of claim 3, wherein the coupling insert comprises a punch (74) forming a fulcrum for the arm against the opposite face of the plate.

6. The capsule of claim 3, wherein the coupling insert comprises a ball or roller bearing (76) forming a journal bearing for the arm against the opposite face of the plate.

7. The capsule of claim 1, wherein the coupling means (62) comprise an articulation with a ball joint (64) integral with the free end of the plate, cooperating with a seat (66) formed on the link element, or vice versa.

8. The capsule of claim 1, comprising an asymmetric structure, wherein the link element (48) is connected at a central point to the actuation element (44).

9. The capsule of claim 1, comprising a symmetric split structure, with two piezoelectric components (50, 50') and two respective link elements (48, 48') connected to the common actuation element (44) at diametrically opposed points of this actuation element.

10. The structure of claim 1, wherein the plate (50) has a progressively decreasing width from its embedded end (52) to its free end (60).
